# EUROPEAN PATENT SPECIFICATION

(11) **EP 3 476 938 B1**
(45) Date of publication and mention of the grant of the patent: **13.05.2020**
(21) Application number: 17905910.0
(22) Date of filing: 14.09.2017
(51) Int. Cl.: C12N 15/10, C12Q 1/68

(54) **METHOD AND KIT FOR SYNTHESIZING NUCLEIC ACID UNDER CONSTANT TEMPERATURE CONDITIONS**
VERFAHREN UND KIT ZUR SYNTHESE EINER NUKLEINSÄURE UNTER KONSTANTTEMPERATURBEDINGUNG
PROCÉDÉ ET KIT DE SYNTHÈSE D'ACIDE NUCLÉIQUE DANS DES CONDITIONS DE TEMPÉRATURE CONSTANTES

(43) Date of publication of application: 01.05.2019
(73) Proprietor: Zhongke Xinray (Suzhou) Biological Science Technologies Co., Ltd., 215152 Suzhou (CN)
(72) Inventor: DU, Yuguang, Beijing 100190 (CN); MAO, Rui, Beijing 100190 (CN); WANG, Zhuo, Beijing 100190 (CN)
(74) Representative: Rössler, Matthias
(86) International application number: PCT/CN2017/101789
(87) International publication number: WO 2019/051732

(56) References cited:
- CN-A- 1 432 061
- CN-A- 106 636 071
- CN-C- 100 393 875
- US-A1- 2011 008 781
- YU SHERRY JIANG ET AL: "Robust Strand Exchange Reactions for the Sequence-Specific, Real-Time Detection of Nucleic Acid Amplicons", ANALYTICAL CHEMISTRY, vol. 87, no. 6, 17 March 2015 (2015-03-17) , pages 3314-3320, XP055563738, US ISSN: 0003-2700, DOI: 10.1021/ac504387c
- YAN DU ET AL: "A Sweet Spot for Molecular Diagnostics: Coupling Isothermal Amplification and Strand Exchange Circuits to Glucometers", SCIENTIFIC REPORTS, vol. 5, no. 1, 8 June 2015 (2015-06-08), XP055563736, DOI: 10.1038/srep11039
- GRZEGORZ WOZNIAKOWSKI ET AL: "Polymerase cross-linking spiral reaction (PCLSR) for detection of African swine fever virus (ASFV) in pigs and wild boars", SCIENTIFIC REPORTS, vol. 7, no. 1, 15 February 2017 (2017-02-15), XP055563721, DOI: 10.1038/srep42903
- WEI LIU ET AL: "Polymerase Spiral Reaction (PSR): A novel isothermal nucleic acid amplification method", SCIENTIFIC REPORTS, vol. 5, no. 1, 29 July 2015 (2015-07-29), XP055563728, DOI: 10.1038/srep12723
- RUI MAO ET AL: "Competitive annealing mediated isothermal amplification of nucleic acids", THE ANALYST, vol. 143, no. 3, 1 January 2018 (2018-01-01), pages 639-642, XP055563622, UK ISSN: 0003-2654, DOI: 10.1039/C7AN01569K
- JIAO, WENQIANG et al.: "Principle and Application of Reverse Transcription Loop-mediated Isothermal Amplification for Detection of Pathogenic microorganisms", Shengwu-jishu-tongbao = Biotechnology bulletin, no. 9, 30 September 2009 (2009-09-30), XP009513123, ISSN: 1002-5464, DOI: 10.13560/j .cnki .bio tech.bul l .1985.2009.09.027
- WANG, YI et al.: "Multiple Endonuclease Restriction Real-Time Loop-Mediated Isothermal Amplification", Journal of Molecular Diagnostics, vol. 17, no. 4, 31 July 2015 (2015-07-31), XP055562524, ISSN: 1525-1578
- ZHAO, YONGXI et al.: "Isothermal Amplification of Nucleic Acids", Chemical Reviews, vol. 115, no. 22, 9 November 2015 (2015-11-09), XP055562529, ISSN: 0009-2665

<110> Zhongke Xinrui (Suzhou) Biotechnology Co Ltd.
<120> Method and kit for synthesizing nucleic acid under constant
   temperature condition
<130> 2017
<160> 17
<170> PatentIn version 3.3
<210> 1
   <211> 42
   <212> DNA
   <213> Synthetic primer sequence
<400> 1
   gatcggacga gatcgggcgt agcttacggc cataccagcc tg 42
<210> 2
   <211> 41
   <212> DNA
   <213> Synthetic primer sequence
<400> 2
   tacgcccgat ctcgtccgat ccccgaccct gcttagcttc c 41
<210> 3
   <211> 122
   <212> DNA
   <213> Pangasianodon hypophthalmus 5S rRNA
<400> 3
   gcttacggcc ataccagcct gaatacgccc gatctcgtcc gatctcggaa gctaagcagg      60
   gtcgggcctg gttagtactt ggatgggaga ccgcctggga ataccaggtg ctgtaagctt     120
   tt                                                                    122
<210> 4
   <211> 42
   <212> DNA
   <213> Synthetic primer sequence
<400> 4
   cacaggcaac aagaaaagtg tcatttgtga ctatggcctt cg 42
<210> 5
   <211> 42
   <212> DNA
   <213> Synthetic primer sequence
<400> 5
   gacacttttc ttgttgcctg tgggagtagt gggctcgtag ac 42
<210> 6
   <211> 301
   <212> DNA
   <213> MERS-orf1a
<400> 6
   tctagacacg tgtaatacga ctcactatag gtatgtgata atcttacaag ccactaaatt      60
   tactttgtgg aactacttgt ttgagactat tccatatgcc acacagttgt tcccactctt     120
   atttgtgact atggccttcg ttatgttgtt ggttctcgag aaacacaaac acaccttttt     180
   gacacttttc ttgttgcctg tggctatttg tttgacttat gcaaagctta acatagtcta     240
   cgagcccact actcccattt cgtcagcgct gattgcagtt gcaaattggg ttaacgaatt     300
   c                                                                     301
<210> 7
   <211> 301
   <212> RNA
   <213> MERS-orfla
<400> 7
   ucuagacacg uguaauacga cucacuauag guaugugaua aucuuacaag ccacuaaauu 60
   uacuuugugg aacuacuugu uugagacuau uccauaugcc acacaguugu ucccacucuu 120
   auuugugacu auggccuucg uuauguuguu gguucucgag aaacacaaac acaccuuuuu 180
   gacacuuuuc uuguugccug uggcuauuug uuugacuuau gcaaagcuua acauagucua 240
   cgagcccacu acucccauuu cgucagcgcu gauugcaguu gcaaauuggg uuaacgaauu 300
   c 301
<210> 8
   <211> 18
   <212> DNA
   <213> Synthetic primer sequence
<400> 8
   attcccacac agttgttc 18
<210> 9
   <211> 19
   <212> DNA
   <213> Synthetic primer sequence
<400> 9
   tgcaatcagc gctgacgaa 19
<210> 10
   <211> 19
   <212> DNA
   <213> Synthetic primer sequence
<400> 10
   ggttaaacac aaacacacc 19
<210> 11
   <211> 20
   <212> DNA
   <213> Synthetic primer sequence
<400> 11
   gcataagtca aacaaatagc 20
<210> 12
   <211> 41
   <212> DNA
   <213> Synthetic primer sequence
<400> 12
   cgaaagcata ccatggtggg agacactata acatttgaag c 41
<210> 13
   <211> 38
   <212> DNA
   <213> Synthetic primer sequence
<400> 13
   caccatggta tgctttcggt gaactggagc atctgatg 38
<210> 14
   <211> 356
   <212> DNA
   <213> Influenza virus H1N1
<400> 14
   tttcagttgg atcgtcaaaa tacaaccgaa gattcgctcc ggaaatagca gctagaccta 60
   aagttagagg acaggcaggc agaatgaact attattggac actattagac caaggagaca 120
   ctataacatt tgaagccact gggaatttga tagcaccatg gtatgctttc gcattgaata 180
   aggggtctga ctctggaatt ataacatcag atgctccagt tcacaattgt gacacaaggt 240
   gccaaacccc tcatggggct ttgaacagca gccttccttt tcagaatgta catcctatca 300
   ctattggaga atgtcccaaa tacgtcaaga gcaccaaact aagaatggca acagga 356
<210> 15
   <211> 40
   <212> DNA
   <213> Synthetic primer sequence
<400> 15
   aggtcgggga agaactgtca cctgtacgag gtgatgcagc 40
<210> 16
   <211> 39
   <212> DNA
   <213> Synthetic primer sequence
<400> 16
   gacagttctt ccccgacctc cgccacgatc acgtacttg 39
<210> 17
   <211> 322
   <212> DNA
   <213> Cyprinid herpes virus-3
<400> 17
   cgggcgacgc tgcatcgccg tcaagcacgc catagaccag cgctacaccg aagagtccaa     60
   ggtggccatg cacagcggcg cgacctaccc ggccatctcc gcgggttacc tgtacgaggt    120
   gatgcagcgt ctggaggaat acgacgccgt ggccgtcgac gagggacagt tcttccccga    180
   cctctacgag ggagtcgtgc agctgctgac cgcgggcaag tacgtgatcg tggcggcgct    240
   ggacggggac tttatgcagc agcccttcaa gcaggtgacg gcgttggtgc ccatggcgga    300
   caagctggac aagctgacgg cg                                             322

## Description

### Technical Field

The present invention relates to the field of genetic engineering technology, specifically to a method for synthesizing a nucleic acid under constant temperature condition, and in particular to a synthetic method capable of forming a special structural nucleic acid composed of a specific nucleotide sequence, and a method useful for amplifying a nucleic acid based on the specific nucleic acid sequence.

### Background Art

The genetic information carried by organisms is the most fundamental difference between organisms, and analytical methods based on complementarity of nucleotide sequences can directly analyze the genetic characteristics carried by genes. This analysis is a very powerful method for identifying genetic diseases, cancer, microorganisms and the like. When the target gene content in a sample is very low, it is generally difficult to detect, so it is necessary to amplify the target gene or amplify the detection signal thereof. As a method for amplifying the target gene, the PCR method is considered to be the most classical method (Saiki, Gelfand et al. 1988), and is also the most commonly applied technique for in-vitro nucleic acid sequence amplification. The exponential amplification result of this method makes it highly sensitive, and has established its position in the detection field by molecular biological method, and there have been a series of mature products after several decades of development. In addition, the amplification products are recyclable and thus widely used as an important tool to support genetic engineering techniques such as gene cloning and structure determination. However, the PCR method obviously has the following problems: in the actual operation, a specialized program temperature control system must be used; the exponential rise of the amplification reaction causes it difficult to be quantified; and the sample and the reaction solution are susceptible to external contamination, and the false positive problem is more prominent.

For example, once a complementary strand is accidentally mis-synthesized in the PCR, the product runs as a template in the following reactions, which will result in erroneous results. In practical applications, if the primer end has only one different base, it will be very difficult to strictly control the PCR, so the specificity must be improved to make the PCR more suitable for the detection of SNPs. On the other hand, scientists have also developed techniques for synthesizing a nucleic acid under constant temperature conditions compared to cumbersome program temperature control process for synthesizing a nucleic acid (Zhao, Chen et al. 2015), mainly including the following: nucleic acid sequence-based amplification (NASBA), rolling circle amplification (RCA), strand displacement amplification (SDA), loop-mediated isothermal amplification (LAMP), helicase-dependent amplification (HDA), and recombinase polymerase amplification (RPA).

NASBA, also known as TMA (Transcription-Mediated Amplification), does not require complex temperature control. The method is characterized by using a target RNA as a template by a DNA polymerase and adding a probe linked with a T7 promoter to achieve the synthesis, allowing a second probe to enter into the double strands to generate a product, and then using the generated double-stranded DNA as a template to perform transcription and amplification by T7 RNA polymerase to obtain a large amount of RNA product. NASBA requires a heat denaturation step until double stranded DNA is formed, but the subsequent transcription reaction is carried out under isothermal conditions by T7 RNA polymerase. The combination of a variety of enzymes such as reverse transcriptase, RNase H, DNA polymerase and T7 RNA polymerase must be used, but the combination of a variety of enzymes is disadvantageous for cost. At the same time, due to the complex set of various enzyme reaction conditions, it is very difficult for this method to be generalized as a general analytical method.

RCA (Rolling Circle Amplification) is intended to mimic the rolling circle replication process of circular DNA in microorganisms. For a circular single-stranded DNA template, the primer combined with the template can only achieve the amplification of circular nucleic acids in the original method. In order to make the method universal for linear DNA amplification, it is shown that a single-stranded DNA having a series of nucleotide sequences complementary to that of a padlock probe or a circular probe can be synthesized continuously in the presence of target nucleotides by a padlock probe or a circular probe complementary single-stranded DNA (Lizardi, Huang et al. 1998). There is also the problem of requiring a variety of enzymes in this method. Moreover, the initiation of complementary strand synthesis depends on the reaction joining two adjacent regions, and the specificity thereof is substantially the same as that in the LCR.

The Strand Displacement Amplification (SDA) method is also known as a method for amplifying a template DNA having a sequence complementary to a target sequence (Zhang, Cui et al. 1992). The SDA method uses a specific DNA polymerase to synthesize a complementary strand starting from the 3'-side complementary primer of the target nucleotide sequence to displace the double-stranded 5'-side sequence. Since the newly synthesized complementary strand displaces the 5'-side double strands, the technique is referred to as SDA method. In the SDA method, the restriction enzyme recognition sequence is inserted as a primer into an annealing sequence, which is capable of removing the temperature change step necessary in the PCR method. That is, the 3'-OH group is supplied by restriction enzyme-generated nicks to serve as a synthetic starting point of the complementary strand, and the first synthesized complementary strand releases a single strand by strand displacement synthesis, and then is used again as a template for the following complementary strand synthesis. However, SDA amplification products differ from natural nucleic acid in structures and have limitations on the use of restriction enzymes for cleaving or the application of the amplification products to gene cloning. This is also the main reason for the high cost. In addition, when the SDA method is applied to an unknown sequence, the nucleotide sequence the same as the restriction enzyme recognition sequence introduced into a nick may exist in the region to be synthesized, thus possibly preventing the synthesis of the complete complementary strand.

Helicase-dependent Isothermal DNA Amplification (Helicase-dependent Isothermal DNA Amplification , HDA) is a novel nucleic acid constant temperature amplification technique invented by researchers from NEB Corporation in the United States in 2004 (Vincent, Xu et al. 2004). This technique mimics the natural process of DNA replication in natural organisms, uses a helicase to unwind DNA double-strands under constant temperature conditions, while a single-stranded DNA-binding protein (SSB) is used to stabilize the unwound single strand and provide the primer with a binding template, and then a complementary strand is synthesized by the catalysis of a DNA polymerase. The newly synthesized double strands are unwound into single strands under the action of the helicase, and enter the above-mentioned cyclic amplification reaction as a template for the next round of synthesis, finally achieving an exponential growth of the target sequence.

Recombinase Polymerase Amplification (Recombinase Polymerase Amplification, RPA) has its main point lying in that the protein-DNA complex formed by the combination of the recombinase with the primer can find a homologous sequence in the double-stranded DNA. Once the primer localizes the homologous sequence, strand displacement DNA polymerase mediates the formation of the strand exchange reaction and initiates DNA synthesis, exponentially amplifying the target region on the template. The replaced DNA strand binds to a single-stranded binding protein (SSB), to prevent further replacement. In this system, a synthetic event is initiated by two opposing primers. The entire process proceeds very quickly and generally obtains amplification products of a detectable level within ten minutes. However, during the whole process, it is necessary to screen primers which can bind to the recombinase and has good specificity, and the use of three enzymes will greatly increase the cost thereof, and it is more difficult to design the primers.

The core of LAMP technology (Notomi, Okayama et al. 2000) is to design four specific primers for six regions on the target gene and use a high-activity strand-replacement DNA polymerase, so that strand-displacement DNA synthesis is constantly self-circulating. The technique can achieve 109-1010 times amplification within 15-60 minutes, and the reaction can produce a large amount of amplification product, namely white magnesium pyrophosphate precipitate, and the presence or absence of white precipitate can be visually observed to determine whether or not the target gene exists, and Japan Rongyan Corporation has also developed a turbidity meter in a targeted manner to achieve real-time monitoring of the amplification reaction. In addition to high specificity and high sensitivity, the LAMP method also has the following advantages: it has very simple operation, low instrument requirements during the application phase with one simple thermostat capable of realizing the reaction, and very simple result detection by directly visually observing the white precipitate or green fluorescence, and it is a method suitable for on-site and basic level rapid detection. One limitation is that, since the characteristics of the method such as high specificity and sensitivity and so on rely on the nature of the four primers, the acquisition of optimal primers usually requires sequence alignment, on-line primer design, primer screening and specificity test, but this process is very cumbersome. At the same time, the target gene fragment required for LAMP technology is also larger, so it is difficult to apply to short-strand nucleic acids. For example, 5S rRNA is composed of approximately 120 nucleotide units and is an important structural and functional ribosomal subunit in all living bodies. 5S rRNA has high conservation and thus is often selected as a marker for molecular detection. Since the nucleic acid sequence of 5S rRNA is short, the detection of this marker is now generally carried out by a PCR method. Isothermal amplification of nucleic acids has the advantage of simplicity and quickness, but it is difficult to apply to the detection of 5S rRNA. Taking loop-mediated constant temperature amplification (LAMP) as an example, LAMP primers are composed of F3, B3, FIP and BIP, and the nucleic acid sequence required for designing these primers are about 120 bases, let alone designing the interval between F2 and B2, and the severe conditions for LAMP primer design are difficult to meet, so it cannot be applied to the detection of 5S rRNA.

### Description of the Invention

An object of the present invention is to provide a method for synthesizing a nucleic acid, which is based on a new principle inspired by the common double helix structure of DNA, molecular beacon probe and LAMP, redesigns and plans the working mode of primers based on PCR primers, forms a competitive stem-loop initial amplification structure, designs the formation process of the structural loop, and features the optional use of an outer primer. More specifically, a novel low-cost method for efficiently synthesizing a nucleic acid by means of sequences is provided. That is, an object of the present invention is to provide a method for accomplishing nucleic acid synthesis and amplification by a single enzyme and under isothermal conditions. Another object of the present invention is to provide a method for synthesizing a nucleic acid, the method can rapidly synthesize a high-specificity nucleic acid which is difficult to achieve by modifying an existing nucleic acid synthesis reaction principle, and the present invention also provides a method for amplifying a nucleic acid by the synthesizing method. One advantage of the present invention is that, rapid amplification of a gene can be achieved using a single enzyme constant temperature system for short-strand nucleic acid fragments (an ideal fragment can be only 60 bp, which is shorter than the minimum ideal fragment 120 bp of LAMP). The invention utilizes a polymerase to catalyze the strand displacement complementary strand synthesis without complicated temperature control, being beneficial to the synthesis of nucleic acids. The DNA polymerase is an enzyme used in the methods such as SDA, RCA, LAMP and the like.

The present inventors have improved the supply of 3'-OH in the known method, and as a result, it is found that a 3'-OH structure can be provided by using an oligonucleotide having a specific structure without any additional enzyme reaction, thereby obtaining the present invention. That is, the present invention relates to a method for synthesizing a nucleic acid, a method for amplifying a nucleic acid by the method for synthesizing a nucleic acid, and a kit for synthesizing a nucleic acid using the method.

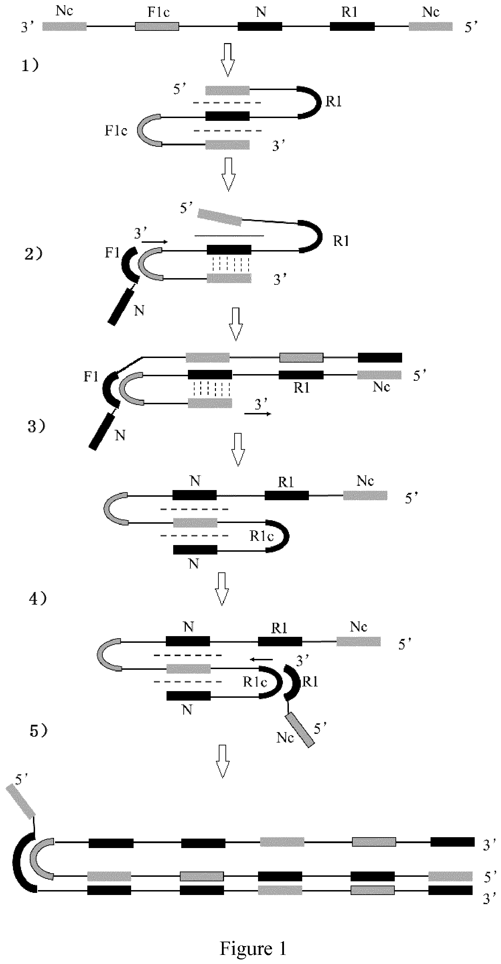

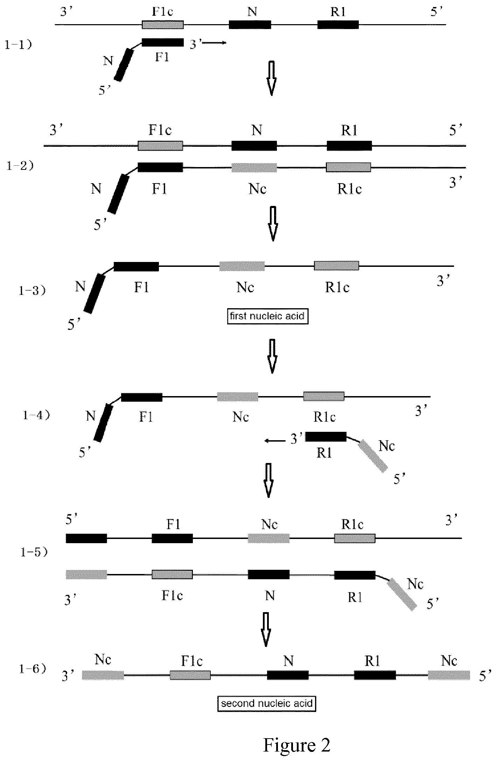

The specific technical solutions of the present invention are as follows:
The invention provides a method for synthesizing a nucleic acid under constant temperature conditions, comprising the steps of:
1) a step of providing a nucleic acid, wherein the nucleic acid has an Nc region that can anneal to an N region on the same strand at each of the 5' end and the 3' end; a loop can be formed when the Nc region at the 3' end anneals to the N region, and the loop comprises an F1c region capable of base pairing; a loop can be formed when the Nc region at the 5' end anneals to the N region, and the loop comprises an R1 region capable of base pairing; and the Nc regions at the 5' end and the 3' end of the nucleic acid have a competitive relationship in annealing to the N region on the same strand;
2) annealing a first oligonucleotide I to the F1c region of the nucleic acid provided in the step 1), and then performing the synthesis step using an F1 region of the first oligonucleotide I as a synthesis starting point; wherein the first oligonucleotide I comprises an N region and the F1 region;
3) using the nucleic acid provided in the step 1) as a template to synthesize its own complementary strand with the 3' end of the Nc region which has annealed to the N region as a synthetic starting point; and referring to the nucleic acid sequence after the synthesis is finished as nucleic acid A;
4) annealing a second oligonucleotide II to an R1c region of the nucleic acid A provided in the step 3), and then performing the synthesis step using an R1 region of the second oligonucleotide II as a synthesis starting point; wherein the second oligonucleotide II comprises the R1 region and an Nc region;
5) using the nucleic acid A provided in the step 3) as a template to synthesize its own complementary strand with the 3' end of the N region which has annealed to the Nc region as a synthetic starting point, and obtaining a nucleic acid having a head-to-tail complementary nucleotide sequence on one strand thereof, and the nucleic acid strand having alternately linked complementary nucleotide sequence regions thereon;
wherein the nucleic acid in the step 1) comprises the steps of:
1-1) an annealing step, annealing the first oligonucleotide I to the F1c region of the template, wherein the 3' end of the template comprises an F1c region and an N region located at the 5' side of the F1c region, and the 5' end of the template comprises an R1 region, wherein the first oligonucleotide I comprises an N region and an F1 region, and the N region is linked with the 5' side of the F1 region, wherein,
   F1 region: a region having a nucleotide sequence complementary to that of the F1c region,
   N region: a region of a nucleotide sequence complementary to that of the Nc region;
1-2) synthesizing a first nucleic acid using the F1 region of the first oligonucleotide I as a synthesis starting point; wherein the first nucleic acid has a nucleotide sequence complementary to that of the template, the 5' end of the first nucleic acid has an N region that can anneal to the Nc region on the same strand, and a stem-loop can be formed by annealing the Nc region to the N region;
1-3) displacement obtaining the first nucleic acid synthesized in the step 1-2) by using a polymerase to catalyze a strand displacement reaction;
1-4) an annealing step, annealing the second oligonucleotide II to the R1c region of the first nucleic acid obtained in the step 1-3), wherein the second oligonucleotide II comprises an R1 region and an Nc region, and the Nc region is linked with the 5' side of the R1 region; wherein
   R1 region: a region having a nucleotide sequence complementary to that of the R1c region,
   Nc region: a region of a nucleotide sequence complementary to that of the N region;
1-5) synthesizing a second nucleic acid using the R1 region of the second oligonucleotide II as a starting point for synthesis; and
1-6) obtaining the nucleic acid of the step 1) by using a polymerase to catalyze a strand displacement reaction to displace the second nucleic acid.
   Referring to Figure 1, it illustrates the steps corresponding to the above synthesis method of a nucleic acid according to the present invention;
   Referring to Figure 2, it illustrates the steps corresponding to the second nucleic acid (i.e., the nucleic acid described in the step 1)) in the present invention.

Preferably, the template in the step 1-1) is RNA, and the first nucleic acid in the step 1-2) is synthesized by an enzyme having reverse transcriptase activity.

The present invention is suitable for various DNAs and RNAs, such as DNA and RNA of various animal and plant cells, bacteria and viruses, and the like. For example, the present invention is used for cDNA and RNA detection of HI gene and N1 gene of H1N1 virus; used for detection of cDNA and RNA of MERS-CoV virus, such as orfla, orflb segments of the RNA; and used for DNA detection of *Cyprinidae herpes* virus III, etc.

Preferably, the nucleic acid fragments of the F1c region, the N region and the R1 region are all 15-60 bp. Further preferably, the nucleic acid fragments of the F1c region, the N region and the R1 region are all 20 bp.

The method for synthesizing a nucleic acid under constant temperature conditions further comprises: the nucleic acid strand obtained in the step 5) being capable of autonomously pairing for infinite extension, and the 3' end of Nc region on the nucleic acid strand being paired with the complementary segment N region on the strand to act as a starting point of synthesis and allow the nucleic acid strand to continuously extend using itself as a template.

In the method for synthesizing a nucleic acid according to the present invention, the synthesized nucleic acid refers to a nucleic acid having a head-to-tail complementary nucleotide sequence on one strand thereof.

In the method for synthesizing a nucleic acid according to the present invention, the constant temperature refers to that the entire reaction process is carried out within a temperature range of from 60 to 65°C.

In the method for synthesizing a nucleic acid according to the present invention, preferably, the nucleic acid amplification is accelerated by a method of introducing the accelerating primer X2 and/or Xin; wherein, X2 is a segment located at the 5' side of the R1 region and the F1 region of the complementary strand of the original nucleic acid, and Xin is a middle segment located from the F1c region to the N region and the Nc region to R1c region. The polymerase used in the polymerase catalytic strand displacement reaction of the present invention is one or more of Bst DNA polymerase, Bca (exo-) DNA polymerase, DNA polymerase I Klenow fragment, Vent DNA polymerase, Vent (Exo-) DNA polymerase (Vent DNA polymerase lacking exonuclease activity), Deep Vent DNA polymerase, Deep Vent (Exo-) DNA polymerase (Deep Vent DNA polymerase lacking exonuclease activity), Φ29 phage DNA polymerase and MS-2 phage DNA polymerase. Among them, Bst DNA polymerase or Bca (exo-) DNA polymerase is preferably used.

In the method for synthesizing a nucleic acid according to the present invention, a melting temperature adjusting agent can be added into the polymerase catalytic strand displacement reaction. Preferably, the melting temperature adjusting agent is a betaine, and further preferably, the concentration of the betaine in the reaction solution is 0.2 to 3.0 M.

A reference embodiment of this application is a kit for synthesizing a nucleic acid under constant temperature conditions of the present invention is characterized in that the kit comprises:
a first oligonucleotide I, comprising an F1 region and an N region, the N region being linked with the 5' side of the F1 region, wherein,
   F1 region: a region having a nucleotide sequence complementary to that of the F1c region, and
   N region: a region of a nucleotide sequence complementary to that of the Nc region;
a second oligonucleotide II, comprising an R1 region and an Nc region, the Nc region being linked with the 5' side of the R1 region, wherein,
   R1 region: a region having a nucleotide sequence complementary to that of the R1c region, and
   Nc region: a region of a nucleotide sequence complementary to that of the N region;
a DNA polymerase catalyzing a strand displacement type complementary strand synthesis reaction, and
a nucleotide that serves as the substrate of the DNA polymerase.

The kit described in the present application, wherein, the kit further comprises a detection reagent for detecting the product of a nucleic acid synthesis reaction.

The kit described herewith, wherein the kit further comprises the acceleration primer X2 and/or Xin, wherein X2 is a segment located at the 5' side of the R1 region and the F1 region of the complementary strand of the original nucleic acid, and Xin is a middle segment located from F1c region to N region and the Nc region to the R1c region.

The kit described herewith, wherein, the DNA polymerase is one or more of Bst DNA polymerase, Bca (exo-) DNA polymerase, DNA polymerase I Klenow fragment, Vent DNA polymerase, Vent (Exo-) DNA polymerase, Deep Vent DNA polymerase, Deep Vent (Exo-) DNA polymerase, Φ29 phage DNA polymerase, and MS-2 phage DNA polymerase. Among them, Bst DNA polymerase or Bca (exo-) DNA polymerase is preferably used.

The kit described above may be used in synthesizing a nucleic acid or detecting a target nucleotide sequence in a sample.

Based on the method for synthesizing a nucleic acid according to the present invention, a method for detecting a target nucleotide sequence in a sample is described, and comprises performing amplification by the method for synthesizing a nucleic acid of the present invention using a target nucleotide as a template, and observing whether or not an amplification product is generated.

A probe comprising a nucleotide sequence complementary to that of the formed stem-loop structure is added into the above amplification product, and the hybridization between the two is further observed. It is also possible to label the probe on the particles and observe the aggregation reaction which occurs by hybridization. The amplification method can be carried out in the presence of a nucleic acid detection reagent, and it is observed whether or not an amplification product is generated based on a change in the signal of the detection reagent.

Based on the method for synthesizing a nucleic acid according to the present invention, a method for detecting a mutation of a target nucleotide sequence in a sample can be further described and comprises performing amplification by the method for synthesizing a nucleic acid according to the present invention using a target nucleotide as a template. In the method, the mutation as an amplification object in the nucleotide sequence hinders the synthesis of any complementary strand constituting the amplification method, thereby detecting the mutation.

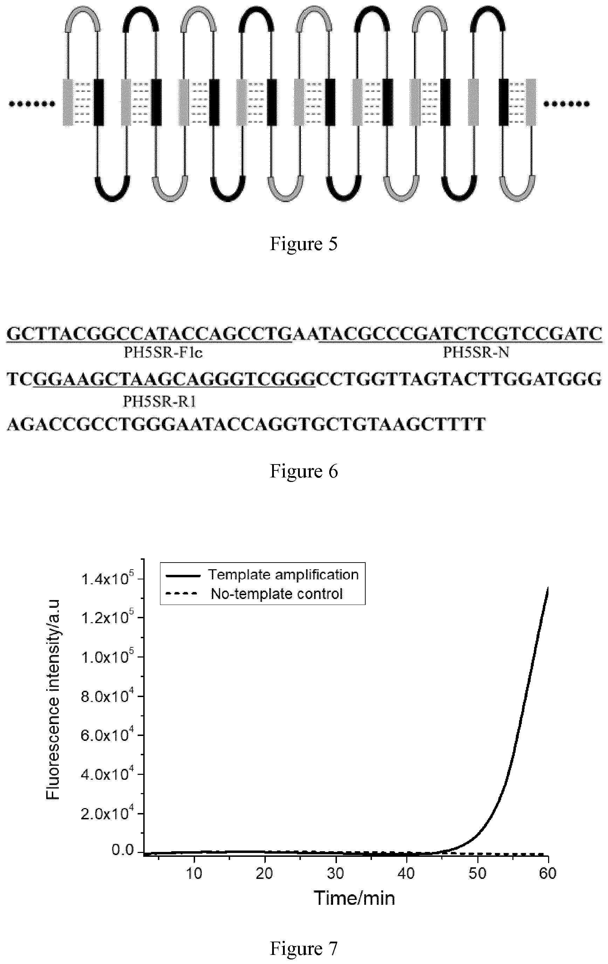

An object of the synthesis of the present invention is a single-stranded nucleic acid capable of forming a nucleotide sequence of a competitive stem-loop structure, and the nucleic acid refers to a nucleic acid taking a target nucleic acid sequence as a mutually complementary nucleic acid sequence alternately linked into a single strand in a certain order. The nucleic acid synthesized by the present invention consists essentially of mutually complementary strands linked by a stem-loop structure. Referring to Figure 5, it is a schematic illustration of an ideal amplification nucleic acid product formed by the synthetic method of the present invention.

In general, a strand that cannot be separated into two or more molecules when paired bases are separated is referred to as a single strand regardless of whether it partially involves base pairing. The complementary nucleotide sequences in the same strand can form base pairing, and the present invention can obtain a product of intramolecular base pairing by allowing base pairing of nucleic acids having a nucleotide sequence head-to-tail linked into a single strand in the same strand, and the product contains a region that constitutes distinct double strands and a loop that does not involve base pairing.

That is, the nucleic acid of the present invention having a nucleotide sequence of a competitive stem-loop structure can be defined as a single-stranded nucleic acid, which comprises therein complementary nucleotide sequences capable of annealing in the same strand. A nucleotide having complementary nucleotide sequences can be annealed into a loop that does not involve base pairing. The loop-forming sequence can be any nucleotide sequence. The loop-forming sequences are capable of base pairing to initiate the synthesis of a complementary strand for displacement. Sequences different from the nucleotide sequences located in other regions are preferentially provided to obtain specific annealing.

The substantially identical nucleotide sequence in the present invention is defined as follows: when a complementary strand synthesized by using a certain sequence as a template anneals to a target nucleotide sequence to supply a starting point for synthesizing a complementary strand, the certain sequence is substantially identical to the target nucleotide sequence. For example, a sequence substantially identical to F1 not only fully includes the sequences identical to F1, but also includes a nucleotide sequence that can serve as a template, and the template can give a nucleotide sequence annealing to F1 and can serve as a starting point for synthesizing a complementary strand. The term "anneal" in the present invention refers to the formation of a nucleic acid of complementary structure by base pairing according to Watson-Crick's law. Therefore, even if the nucleic acid strand constituting the base pairing is single-stranded, annealing would occur if the complementary nucleotide sequences in the molecule are base-paired. By base-pairing, a nucleic acid constitutes a double-stranded structure, so that the meanings expressed by annealing and hybridization in the present invention have overlapping portions.

The pair number of the nucleotide sequences constituting a nucleic acid of the present invention is at least 1. In the desired model of the present invention, the pair number of the nucleotide sequences can be an integral multiple of 1. In this case, the pair number of the complementary nucleotide sequences of the constituent nucleotides of the present invention has no upper limit theoretically, and when the synthetic product nucleic acid of the present invention consists of a plurality of sets of complementary nucleotide sequences, the nucleic acid is composed of repeated identical nucleotide sequence.

The single-stranded nucleotide of the nucleotide sequence having a competitive stem-loop structure synthesized by the present invention has a structure different from that of a naturally occurring nucleic acid, and it is generally known that a nucleic acid derivative can be synthesized if a nucleotide derivative is used as a substrate when synthesizing a nucleic acid by nucleic acid polymerase action. The used nucleotide derivative includes radioisotope-labeled nucleotides or binding ligand-labeled nucleotide derivative, such as biotin or digoxin. These nucleotide derivatives can be used to label product nucleic acids. Alternatively, if the substrate is a fluorescent nucleotide, the product nucleic acid may be a fluorescent derivative.

The synthesis of a nucleic acid having the above structure can be initiated by a DNA polymerase, which has a strand displacement activity, and the Nc region at the 3'-end anneals to the portion of N region on the same strand to further synthesize a complementary strand. There are many reports about the synthesis reaction of complementary strands, wherein a helical loop is formed and the spiral loop sequence itself is used as a template, or a hairpin loop is formed and the hairpin loop sequence itself is used as a template, and the present invention provides a competitive hairpin loop with a region capable of partially base-pairing and has new features of utilizing this region when synthesizing complementary strands. By using this region as a starting point for synthesis, the complementary strand previously synthesized with the hairpin loop sequence itself as a template is displaced.

The term "nucleic acid" is used in the present invention, and the nucleic acid in the present invention generally includes both DNA and RNA. However, the template for synthesizing a nucleic acid of the present invention is derived from a natural DNA or RNA, the nucleotide of which is partially displaced or modified. Typically, the nucleic acids of the present invention are included in biological samples, including tissues, cells, cultures and secretions of animals, plants or microorganisms, and extracts thereof. Biological samples of the present invention include genomic DNA or RNA of intracellular parasite, such as viruses or mycoplasmas. The nucleic acid of the present invention is generally derived from a nucleic acid contained in the biological sample. For example, cDNA is synthesized from mRNA, a nucleic acid which is amplified based on a nucleic acid derived from a biological sample is a typical example of the nucleic acid of the present invention.

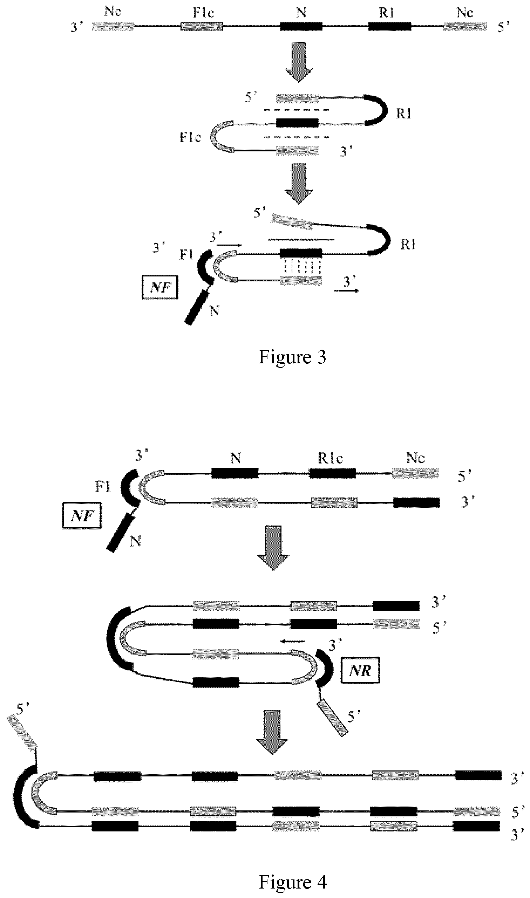

The nucleic acid of the present invention is characterized in that an Nc region is provided at each of the 3'-end and 5'-end and can anneal to the portion of N region on the same strand, and a hairpin ring can be formed by annealing the Nc region to the N region on the same strand, and the nucleic acid can be obtained in various methods, the Nc regions at the 5' end and the 3' end of the nucleic acid have a competitive relationship in annealing to the N region on the same strand. Furthermore, it is possible to extend under the action of DNA polymerase only when the Nc region at the 3'-end anneals to the N region. Referring to Figure 3, it is a schematic illustration of a competitive hairpin structure formed by a single-stranded nucleic acid and the subsequent amplification reactions of the present invention. Figure 4 is a schematic illustration of the subsequent amplification reactions of the nucleic acid. Here, NF in Figures 3 and 4 refers to the first oligonucleotide I, and NR in Figure 4 refers to the second oligonucleotide II.

The terms "identical" and "complementary" as used in constituting the nucleotide sequence features of the oligonucleotides of the present invention are not meant to be absolutely identical and absolutely complementary. That is, a sequence identical to a certain sequence includes a sequence complementary to a nucleotide sequence annealed to a certain sequence. On the other hand, the complementary sequence is a sequence that can be annealed under stringent conditions, providing a 3'-end as a starting point for the synthesis of the complementary strand.

In the present invention, an oligonucleotide is a nucleotide that satisfies two requirements, i.e., must be capable of forming a complementary base pairing and supplying an -OH group at the 3'-end as a starting point for complementary strand synthesis. Therefore, its main strand is not necessarily limited to one linkage of a phosphodiester bond. For example, the main strand of it may be composed of a thiophosphoric acid derivative or it is a peptide nucleic acid based on peptide linkage, and the thiophosphoric acid derivative has O substituted by S. Bases are those bases that are complementary to and paired with each other. There are five naturally occurring bases, namely A, C, T, G and U, and the base may also be an analog such as bromodeoxyuridine. Preferably, the oligonucleotide of the present invention can be used not only as a starting point for synthesis but also as a template for complementary strand synthesis. The term polynucleotide of the present invention includes oligonucleotides. The term "polynucleotide" as used in the present invention has it strand length unlimited, and the term "oligonucleotide" as used herein refers to a nucleotide polymer having a relatively short strand length.

In various nucleic acid synthesis reactions described below, under the given conditions, the oligonucleotide strand of the present invention has such a length that is capable of base pairing with the complementary strand and maintaining certain specificity. Specifically, it consists of 5 to 200 bases, more preferably 10 to 50 base pairs. The known polymerase is identified to have a strand length of at least 5 bases. The polymerase catalyzes a sequence-dependent nucleic acid synthesis reaction. Therefore, the strand length of the annealed portion should be longer than this length. In addition, it is statistically desirable to have a length of 10 bases or longer to obtain target nucleotide specificity. On the other hand, it is difficult to prepare a too long nucleotide sequence by chemical synthesis, so the above strand length is an example of a desired range. The strand length exemplified refers to the strand length of the portion that anneals to the complementary strand. As described below, the oligonucleotides of the present invention may eventually anneal at least to two regions, respectively. Thus, the strand length exemplified herein should be understood as the strand length of each region that constitutes the oligonucleotide.

Furthermore, the oligonucleotides of the present invention may be labeled with known labels. Labels include binding ligands such as digoxin and biotin, enzymes, fluorescent substance, illuminants, and radioisotopes. The technique for replacing a base constituting an oligonucleotide by a fluorescent analog is well known (WO95/05391, Proc. Natl. Acad. Sci. USA, 91, 6644-6648, 1994).

Other oligonucleotides of the present invention may also be bound to a solid phase. Alternatively, any portion of the oligonucleotide may be labeled with a binding ligand, such as biotin, indirectly immobilized by a binding ligand such as immobilized avidin. When the immobilized oligonucleotide is the starting point for synthesis, the synthetic reaction product nucleic acid is captured by the solid phase, which will facilitate its separation. The separated portion can be detected by nucleic acid specific indicators or hybridization with labeled probes. For the nucleic acid product obtained by the present method, the target nucleic acid fragment can be recovered by digesting the product by a restriction enzyme.

The term "template" as used in the present invention refers to a nucleic acid used as a template for the synthesis of a complementary strand. A complementary strand having a nucleotide sequence complementary to that of a template refers to a strand corresponding to the template. However, the relationship between the two is only relative. That is, the synthesized complementary strand can once again function as a template. That is, the complementary strand can also serve as a template.

In the present invention, if the target is RNA, it can be constituted only by adding a reverse transcriptase. That is, using RNA as a template, it is possible to synthesize a complementary strand by annealing the F1 to F1c in the template by a reverse transcriptase. When a reverse transcriptase uses DNA as a template to synthesize a complementary strand, all reactions that synthesize a complementary strand by a reverse transcriptase include the synthesis of a complementary strand that uses R1 annealed to R1c as a starting point for synthesis, and the complementary strand is used as a template in the strand displacement reaction. The mode of obtaining the first single-stranded nucleic acid using RNA as a template as described above is a preferred mode of the present invention. On the other hand, if a DNA polymerase having both strand displacement activity and reverse transcriptase activity, such as Bca DNA polymerase is used, not only the synthesis of the first single-stranded nucleic acid from RNA but also the subsequent reactions with DNA as a template can be similarly carried out by the same enzyme.

The reaction is carried out in the presence of the following components: a buffer solution enabling the enzyme reaction at a suitable pH, an essential salt for annealing or maintaining the enzyme catalytic activity, a medium protecting the enzyme, and a regulator necessary for regulating the melting temperature (Tm). For the buffer solution, it is for example Tris-HCl which has a buffering effect in the neutral or weakly alkaline range. The pH value is adjusted according to the DNA polymerase used. For the salt, appropriate amounts of KCl, NaCl, (NH₄)₂SO₄ and so on are added to maintain the activity of the enzyme and regulate the melting temperature (Tm) of the nucleic acid, and the medium for protecting the enzyme uses bovine serum albumin or carbohydrate. In addition, dimethyl sulfoxide (DMSO) or formamide is generally used as the regulator of the melting temperature (Tm). The annealing of the oligonucleotide is regulated by using the regulator of a melting temperature (Tm) under defined temperature conditions. Moreover, betaine (N,N,N-trimethylglycine) or tetraalkyl ammonium salt is also effective for improving the efficiency of strand displacement by its isostabilization. The desired promotion of nucleic acid amplification by the present invention can be achieved by adding 0.2-3.0 M, preferably 0.5-1.5 M betaine to the reaction solution. Since these melting temperature regulators have the effect of lowering the melting temperature, those suitable stringent and reactive conditions are determined empirically by combining with the salt concentration, the reaction temperature and so on.

An important feature of the present invention is that a series of reactions cannot be carried out unless the positional relationships among many regions are maintained. Due to this feature, non-specific synthetic reactions accompanying non-specific synthesis of complementary strands are effectively prevented. That is, even if a certain non-specific reaction occurs, the possibility of the product serving as a starting material in the amplification step subsequent to the synthesis is reduced, and the progress of the reaction is regulated by many regions, possibly resulting in that in similar nucleotide sequences, a detection system capable of accurately identifying the required product can be constructed arbitrarily.

The nucleic acid synthesized by the present invention is a single strand, and in the case of a single strand, it consists of a complementary nucleotide sequence, most of which is base-paired. By utilizing this feature, the synthesized product can be detected. By carrying out the method for synthesizing a nucleic acid of the present invention, in the presence of a fluorchrome such as ethidium bromide, SYBR Green I, Pico Green or Eva Green, as a double-specific intercalater, it can be observed that the intensity of the fluorescence increases as the product increases. By monitoring the fluorescence intensity, it is possible to track the progress of a real-time synthesis reaction in a closed system. It is also conceivable to apply this type of detection system in the PCR method, but there are many problems because the product signal and the signal of a primer dimer cannot be distinguished and so on. However, when the system is applied to the present invention, the ability of increasing non-specific base pairing is very low, so it is anticipated that high sensitivity and low interference may be simultaneously obtained, and similarly to the application of a double-specific intercalater, the transfer of fluorescent energy can be utilized in the same system to implement the method of the detecting system.

The method for synthesizing a nucleic acid of the present invention is supported by a DNA polymerase catalyzed synthesis reaction of a strand displacement type complementary strand. The reaction step of the unnecessary strand displacement type polymerase is also included during the above reaction. However, in order to simplify the constitutive agent and economical viewpoint, it is advantageous to use a DNA polymerase, and the following enzymes are known as the DNA polymerase. Furthermore, various mutants of these enzymes can be utilized in the scope of the present invention, all of which have sequence-dependent activity and strand displacement activity for complementary strand synthesis. The mutant refers to a mutant including those having only the structure causing the catalytic activity of the enzyme or those modified on catalytic activity, stability or thermostability by, for example, mutation in an amino acid.
Bst DNA polymerase
Bca (exo-) DNA polymerase
DNA polymerase I Klenow fragment
Vent DNA polymerase
Vent (Exo-) DNA polymerase (Vent DNA polymerase lacking exonuclease activity)
Deep Vent DNA polymerase
Deep Vent (Exo-) DNA polymerase (Deep Vent DNA polymerase lacking exonuclease activity)
Φ 29 phage DNA polymerase
MS-2 phage DNA polymerase
OmniAmp DNA polymerase

Among these enzymes, Bst DNA polymerase, Bca (exo-) DNA polymerase, and OmniAmp DNA polymerase are particularly desired enzymes, because they have some degree of thermal stability and high catalytic activity. In a preferred embodiment, the reaction of the present invention can be achieved isothermally, but due to the regulation of the melting temperature (Tm) or the like, it is impossible to always utilize the desired temperature conditions to maintain the stability of the enzyme. Therefore, it is one of the conditions required for the thermal stability of the enzyme. Although isothermal reactions are feasible, thermal denaturation can provide a nucleic acid as an initial template, and in this regard, the use of thermostable enzymes broadens the choice of test program.

Various reagents necessary for synthesizing or amplifying a nucleic acid of the present invention may be pre-packaged and provided in the form of a kit. Specifically, the kit provided by the present invention comprises various oligonucleotides necessary as primers for the synthesis of a synthetic complementary strand and external primers for a displacement reaction, a substrate dNTP for the synthesis of a complementary strand, a DNA polymerase for achieving the synthesis of a strand-replacement complementary strand, a buffer solution for providing suitable conditions for enzymatic reactions, and a medium necessary for the detection of the product of the synthesis reaction. Specifically, in a preferred mode of the present invention, the reagents which are not required to be added during the reaction and thus the reagents which must be provided for the reaction after transferring to the reaction vessel, wherein the reaction can be initiated only by the addition of the sample. A system can detect a reaction product in a container by utilizing a visible light signal or a fluorescent signal. It is not necessary to open and close the container after the reaction. This is very beneficial to the prevention of pollution.

The present invention synthesizes a single-stranded nucleic acid having a nucleotide sequence of a competitive stem-loop structure. The nucleic acid has, for example, the following uses: the first feature is the advantage brought by utilizing a specific structure having a complementary sequence in one molecule, the feature may facilitate detection, i.e., there is a known system for detecting a nucleic acid, wherein its changing signal depends on base pairing with a complementary nucleotide sequence. For example, a detection system that fully utilizes the characteristics of the synthetic product of the present invention can be realized by combining with a method in which a double-strand specific intercalater acts as a detecting reagent as described above. If the product of the synthesis reaction of the present invention undergoes a thermal denaturation in the detection system and returns to the original temperature, intramolecular annealing preferentially occurs, and thus rapid base pairing between the complementary sequences is allowed. If a non-specific product is present, they have no complementary sequence in the molecule such that after separating by thermal denaturation into two or more molecules, they cannot immediately return to the original double strands. Interference accompanying non-specific reactions is reduced by the thermal denaturation step provided prior to detection. If the DNA polymerase used is not resistant to heat, the thermal denaturation step has the meaning of termination of the reaction, thus facilitating the control of the reaction temperature.

A second feature is that competitively forming hairpin loops (i.e., stem-loop structures) capable of base pairing are usually formed. The structure of a competitive hairpin loop capable of base pairing is shown in Figure 3. As seen in Figure 3, the loop consists of a nucleotide sequence (from the 3' to 5' end) F1c, N, R1, Nc, and intramolecular annealing can be carried out to form the hairpin loop.

According to a preferred mode of the present invention, a large number of loops capable of base-pairing are supplied in a single-stranded nucleic acid. This means that a large number of probes can hybridize with one molecule of nucleic acid to allow for high-sensitivity detection. It is therefore possible not only to achieve improved sensitivity but also to achieve a method for detecting a nucleic acid based on a special reaction principle such as aggregation. For example, probes immobilized on fine particles such as polystyrene latex are added into the reaction product of the present invention, and the aggregation of the latex particles is observed to hybridize the product with the probes. The intensity of the aggregation can be highly sensitive and quantitatively observed by optical measurement. Alternatively, the aggregation can be observed by naked eyes, so that a reaction system without an optical measuring device can also be established.

Furthermore, the reaction products of the present invention allow for some bindable markers, in which each nucleic acid molecule can be subjected to chromatographic detection. In the field of immunoassay, the actually applied method is an analytical method using a chromatographic medium using visible detection marker (immunochromatography). The method is based on the principle that an analyte is sandwiched between an antibody immobilized on a chromatographic medium and a labeled antibody, and the unreacted labeled component is eluted. The reaction product of the present invention allows this principle to be applied to nucleic acid analysis. That is, a labeled probe for the loop portion is prepared and immobilized on a chromatographic medium to prepare a capture probe for capture, to allow the analysis in the chromatographic medium. A capture probe having a sequence complementary to that of a loop portion is utilized. Since the reaction product of the present invention has a large number of hairpin loops, the product is combined with a large number of labeled probes to give a visually identifiable signal.

The reaction products of the present invention usually can supply base-paired loop regions, and can broaden various other detection systems. For example, a system that uses a surface cytoplasmic genome to detect the loop portion using a fixed probe is feasible. In addition, more sensitive fluorescence analysis can be performed if the probe of the loop portion is labeled with a double-stranded specific intercalater. Alternatively, the ability to synthesize a nucleic acid of the present invention is actively utilized on the 3'- and 5'- sides to form hairpins capable of base-pairing. For example, a loop is designed to have a common nucleotide sequence between normal and abnormal types, while other loops are designed to make a difference therebetween. It is possible to constitute a characteristic analysis system by confirming the presence of a gene in the common portion by the probe and confirming the abnormal presence in other regions. Since the reaction for synthesizing a nucleic acid of the present invention can also be carried out isothermally, the advantage worth mentioning is that a real-time analysis can be performed by a general fluorophotometer. Until then, the structure of the nucleic acid to be annealed in the same strand is known. However, the nucleic acid obtained by the present invention in the single strand having a head-to-tail annealed looped sequence is novel, and contains a large number of loops which can be paired with other bases.

On the other hand, a large number of loops given by the reaction product of the present invention themselves can be used as probes. For example, in a DNA chip, probes are densely packed in a limited region, but in the technique, the number of oligonucleotides capable of being fixed in a certain region is limited, so that a large number of annealable probes can be high-density immobilized by using the product of the present invention, that is, the reaction product of the present invention can be used as a fixed probe on a DNA chip, and the reaction product after amplification can be immobilized by any technique known in the art, or a fixed oligonucleotide is used as the oligonucleotide for the amplification reaction of the present invention, resulting in the formation of a fixed reaction product. Therefore, by using a fixed probe, a large number of sample DNAs are hybridized in a limited region, and as a result, it is expected to give a high signal value.

### Brief Description of the Drawings

Figure 1 is a schematic illustration of the steps of the method for synthesizing a nucleic acid in accordance with the present invention.
Figure 2 is a schematic illustration of the steps of the process for synthesizing the second nucleic acid in accordance with the present invention.
Figure 3 is a schematic illustration of a competitive hairpin structure formed by a single-stranded nucleic acid and the subsequent amplification reactions of the present invention.
Figure 4 is a schematic illustration of the subsequent amplification reactions of the nucleic acid of the present invention.
Figure 5 is a schematic illustration of an ideal amplification product formed by the synthetic method of the present invention.
Figure 6 is a diagram showing the positional relationship of each nucleotide sequence region corresponding to the PH5SR target nucleotide sequence in Example 1 of the present invention.
Figure 7 is a curve graph showing the real-time fluorescence during the PH5SR target nucleotide sequence DNA amplification process under the action of primers in Example 1 of the present invention.
Figure 8 is a diagram showing the positional relationship of each nucleotide sequence region corresponding to the MERS-orfla target nucleotide sequence in Example 2 of the present invention.
Figure 9 is a photograph showing the result of agarose electrophoresis of the product obtained by the method for synthesizing a single-stranded nucleic acid of the present invention using MERS-orfla as a template in Example 2 of the present invention; wherein, Lane 1: Beyotime 00107 DNA Ladder; and Lane 2: 1 fmol MERS-orf1a dsDNA.
Figure 10 is a photograph showing the result of agarose gel electrophoresis of a restriction enzyme digestion product in Example 3 of the present invention, wherein the product is obtained in Example 2 by the nucleic acid synthesis reaction of the present invention, wherein,
   Lane 1: *XhoI* digestion of purified product
   Lane 2: *HindIII* digestion of purified product
   Lane 3: combined *XhoI* and *HindIII* digestion of purified product
   Lane 4: purified product
   Lane 5: molecular weight marker DNA ladder.
Figure 11 is a curve graph showing the real-time fluorescence during the MERS-orf1a target nucleotide sequence DNA amplification under the action of the primers in Example 4 of the present invention.
Figure 12 is a curve graph showing the real-time fluorescence during the in-vitro transcription RNA amplification of MERS-orf1a target nucleotide sequence under the action of the primers in Example 5 of the present invention.
Figure 13 is a schematic illustration showing the action site of the amplification principle of the target nucleotide with the addition of the acceleration probe in Example 6 of the present invention.
Figure 14 is a curve graph showing the fluorescence intensity of the MERS-orfla system amplification overover the reaction time under the action of different combinations of acceleration probe and primers in Example 6 of the present invention.
Figure 15 is a graph showing the real-time fluorescence curve during DNA amplification of the target nucleotide sequence of the influenza A virus H1 gene under the action of the designed influenza A virus H1 target nucleotide primers in Example 7 of the present invention.
Figure 16 is a graph showing the real-time fluorescence curve during DNA amplification of the target nucleotide sequence of the *Cyprinidae herpes* virus III under the action of the *Cyprinidae herpes* virus III target nucleotide primers in Example 8 of the present invention.

### Detailed Description

The experimental methods used in the following examples are all conventional methods unless otherwise specified, and can be carried out by specifically referring to the specific methods listed in the book Molecular Cloning Experimental Guide (third edition) J. Sambrook, or can be carried out according to the kit and product specifications; and the materials and reagents used in the following examples are commercially available unless otherwise specified.

### Example 1 Amplification of Pangasianodon hypophthalmus 5S rRNA fragment

In the method for synthesizing a nucleic acid under constant temperature conditions proposed in the present invention, the smallest target fragment may be a nucleic acid sequence of only 60 bases. In the example, the 5S rRNA gene (Genbank: HQ681111) of *Pangasianodon hypophthalmus* in the literature (Food Chem. 2011; 129: 1860-4.) was selected as the target gene, and constant temperature application of the target gene was achieved by the method of the present invention.

The nucleic acid of the present invention having a complementary strand linked into a single strand in the form of a helical loop was attempted using *Pangasianodon hypophthalmus* 5S rRNA (abbreviated as PH5SR) (from GenBank: HQ681111) artificially designed and inserted with an enzyme cleavage site as a template. Two primers, PH5SRNF (nucleotide sequence as shown in SEQ ID NO. 1) and PH5SRNR (nucleotide sequence as shown in SEQ ID NO. 2), were used in the experiment. These were designed to be annealed into looped regions by utilizing adjacent stacking phenomena. In addition, these primers were set to high concentrations, preferentially causing annealing of PH5SRNF (or PH5SRNR).

The positional relationship of each region of the target nucleotide sequence is shown in Figure 2. Through the primers PH5SRNF and PH5SRNR, two Nc segments were synthesized at both ends of the target nucleotide PH5SR to compete against each other in forming a hairpin loop with the N segment on the target nucleotide sequence, and the synthesis process is shown in Figure 2. The reaction solution combination for the method for synthesizing a nucleic acid of the present invention by these primers is shown below.

The reaction solution combination was as follows, and the rest was made up with ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U *Bst* DNA Polymerase (NEW ENGLAND Biolabs)
1 X EvaGreen (Biotum)
Primers:
1600 nM PH5SRNF
1600 nM PH5SRNR

Target nucleic acid: PH5SR dsDNA (nucleotide sequence as shown in SEQ ID NO. 3). Referring to Figure 6, it shows the positional relationship of each nucleotide sequence region corresponding to the PH5SR target nucleotide sequence.

ABI StepOne real time PCR reaction temperature was set to be constant at 63°C, and the reaction time was 60 min. The curve of the fluorescence intensity overover the reaction time is shown in Figure 7. Fluorescence detection is applied therein so as to achieve the purpose of real-time monitoring, and the result can be judged in advance by the real-time amplification curve.

### Example 2 Amplification of fragments in MERS-orfla

The nucleic acid of the present invention having a complementary strand linked into a single strand in the form of a helical loop was attempted using MERS-orf1a (from GenBank: KX108946.1) artificially designed and inserted with an enzyme cleavage site as a template. Two primers used in the experiment were Mo1aNF (nucleotide sequence as shown in SEQ ID NO. 4) and Mo1aNR (nucleotide sequence as shown in SEQ ID NO. 5). These were designed to be annealed into looped regions by utilizing adjacent stacking phenomena. In addition, these primers were set to high concentrations, preferentially causing annealing of Mo1aNF (or Mo1aNR).

By the primers Mo1aNF and Mo1aNR, two Nc segments were synthesized at both ends of the target nucleotide MERS-orf1a to compete against each other in forming a hairpin loop with the N segment on the target nucleotide. The reaction solution combination for the method for synthesizing a nucleic acid of the present invention by these primers is shown below.

The reaction solution combination was as follows, and the rest was made up with ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U *Bst* DNA Polymerase (NEW ENGLAND Biolabs)
Primers:
1600 nM Mo1aNF
1600 nM Mo1aNR

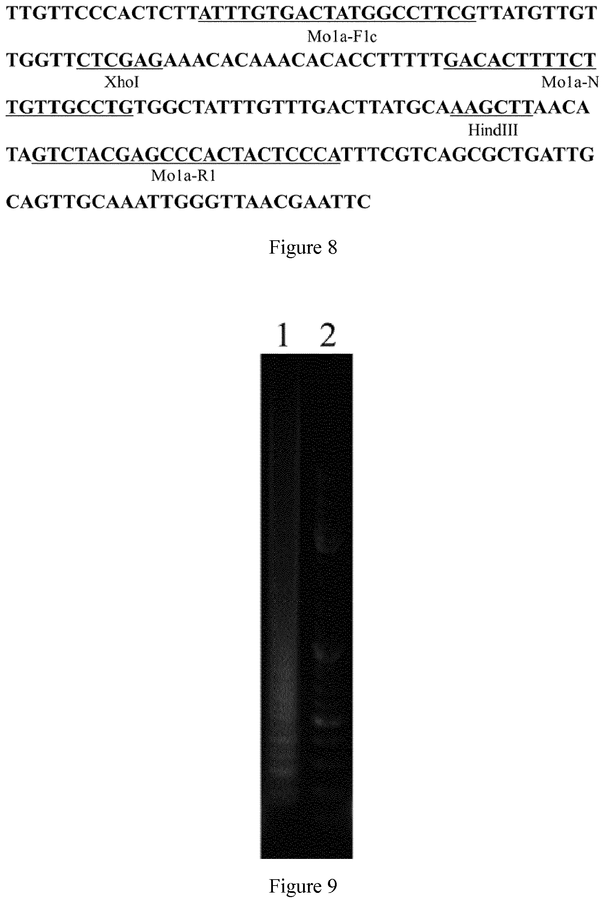

Target nucleic acid: MERS-orf1a dsDNA (nucleotide sequence as shown in SEQ ID NO. 6). Referring to Figure 8, it shows the positional relationship of each nucleotide sequence region corresponding to the MERS-orf1a target nucleotide sequence.

The mixture reacted at 63°C for 1 h, and after the reaction, the reaction was terminated at 80° C over 10 min, and then the mixture was transferred into water pre-cooled with ice.

Verification of the reaction: 1 µL of conventional nucleic acid electrophoresis loading buffer (available from Beyotime DNA ladder) was added to 5 µL of the above reaction solution, and the sample was subjected to electrophoresis on GelRed pre-stained (Biotum) 1% agarose gel (TAE dissolved) for 1 h at 90 mV. Beyotime O0107 DNA Ladder was used as the molecular weight marker. The gel after electrophoresis was used to verify the nucleic acid. The result is shown in Figure 9, which shows that a nucleic acid product having a broad molecular weight distribution is obtained, that is, it is verified that the nucleic acid obtained by the method of the present invention is capable of infinitely self-assembling annealing and extending to obtain an oversized nucleic acid molecule.

### Example 3 Confirmation of the reaction product by digestion of a restriction enzyme

In order to clarify the nucleic acid structure obtained in Example 2 of the present invention having a complementary nucleotide sequence linked into a single strand in a cyclic structure, the product was digested with a restriction enzyme. If a theoretical fragment can be generated by digestion, and at the same time, smeary band pattern and non-electrophoresis band generated at the high molecular weight observed in Example 2 disappears, it can be expected that any of these products is the nucleic acid of the present invention having a complementary sequence alternately linked within a single strand.

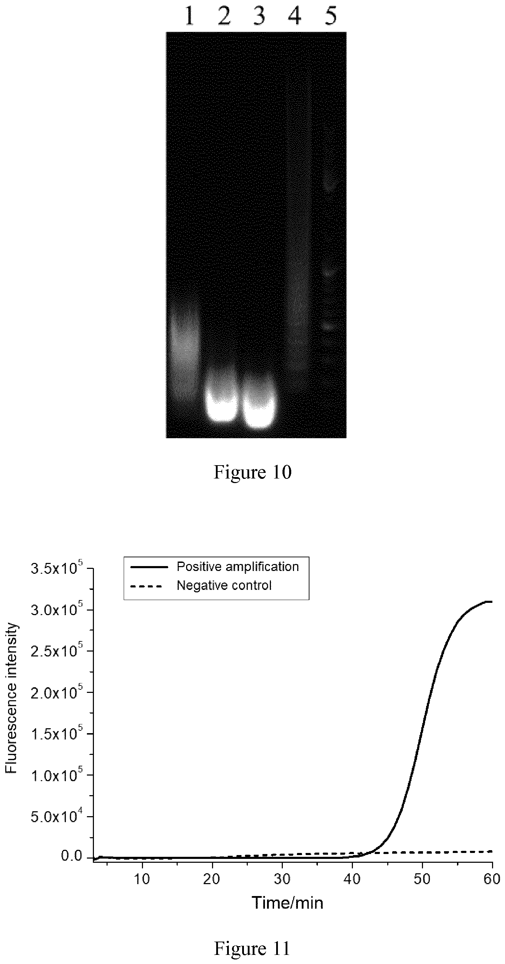

The reaction solution in Example 2 was subjected to treatment with phenol and ethanol precipitation so as to be deposited and purified, and the resulting precipitate was recovered and re-dissolved in ultrapure water, and digested with combination of the two restriction enzymes *HindIII* and *XhoI* at 37°C for 2 h. The sample was subjected to electrophoresis on a GelRed pre-stained (Biotum) 1% agarose gel (TAE dissolution) at 90 mV for 1 h. Beyotime 00107 DNA Ladder was used as the molecular weight marker. The gel after electrophoresis was used to verify the nucleic acid. The result is shown in Figure 10, which shows that the obtained nucleic acid product can be digested by enzyme into small fragments from large fragments, demonstrating that the product is obtained for amplification of the target nucleic acid without non-specific amplification, thereby confirming the specificity of the method of the present invention.

### Example 4 Verification of the reaction product by use of EvaGreen

EvaGreen, similar to SYBR Green I, is a dye with a green excitation wavelength that binds to all dsDNA double helix minor groove regions, and its inhibition on nucleic acid amplification reactions such as PCR is much smaller than that of the latter. When in a free state, EvaGreen emits weak fluorescence, but once bound to double-stranded DNA, the fluorescence is greatly enhanced. Therefore, the fluorescence signal intensity of EvaGreen is related to the number of double-stranded DNAs, and the number of double-stranded DNAs present in the nucleic acid amplification system can be detected based on the fluorescence signal.

The reaction solution combination for the method for synthesizing a nucleic acid of the present invention by the primers MolaNF (nucleotide sequence as shown in SEQ ID NO. 4) and MolaNR (nucleotide sequence as shown in SEQ ID NO. 5) is shown below.

The reaction solution combination was as follows, and the rest was made up with ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U Bst DNA Polymerase (NEW ENGLAND Biolabs)
1 X EvaGreen (Biotum)
Primers:
1600 nM MolaNF
1600 nM MolaNR

Target nucleic acid: MERS-orfla dsDNA (nucleotide sequence as shown in SEQ ID NO. 6). ABI StepOne real time PCR reaction temperature was set to be constant at 63°C, and the reaction time was 60 min. The curve of the fluorescence intensity overover the reaction time is shown in Figure 11. Fluorescence detection is applied therein to achieve the purpose of real-time monitoring, and the results can be judged in advance by the real-time amplification curve.

### Example 5 RNA target gene amplification by use of EvaGreen-based real-time fluorescence

AMV reverse transcriptase can synthesize cDNA using RNA as a template, and the detection of RNA can be achieved by combining with Bst DNA polymerase.

The cDNA was synthesized by the primers MolaNF (nucleotide sequence as shown in SEQ ID NO. 4) and MolaNR (nucleotide sequence as shown in SEQ ID NO. 5) by using RNA as a template, and the reaction solution combination was as follows, and the rest was made up with ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U *Bst* DNA Polymerase (NEW ENGLAND Biolabs)
5 U AMV reverse transcriptase
1 X EvaGreen (Biotum)
Primers:
1600 nM Mo1aNF
1600 nM Mo1aNR

Target nucleic acid: MERS-orf1a RNA (RNA nucleic acid sequence as shown in SEQ ID NO. 7). The MERS-orf1a RNA was obtained by in-vitro transcription of MERS-orf1a (sequence as shown in SEQ ID NO. 6).

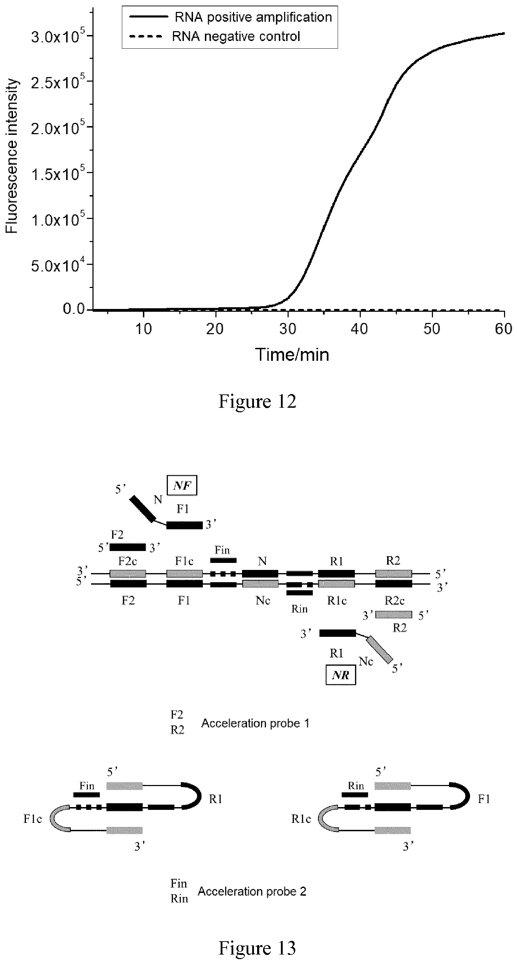

ABI StepOne real time PCR reaction temperature was set to be constant at 63°C, and the reaction time was 60 min. The curve of the fluorescence intensity over the reaction time is as shown in Figure 12. This result indicates that the method is also feasible for RNA detection.

### Example 6 MERS-orf1a dsDNA Target Gene Amplification by use of Acceleration Probes

The reaction solution combination for the method for synthesizing a nucleic acid of the present invention by these primers is shown below.

The acceleration probe combinations were divided into four groups with only the primer combination being different (wherein acceleration probe 1 refers to comprising primers F2 and R2, and acceleration probe 2 refers to comprising primers Fin and Rin):
a, no acceleration probe 1, no acceleration probe 2
b, acceleration probe 1, no acceleration probe 2
c, no acceleration probe 1, acceleration probe 2
d, acceleration probe 1, acceleration probe 2

Referring to Figure 13, it is a schematic illustration showing the action site of the amplification principle of target nucleotide with the addition of acceleration probes.

The reaction solution combination was as follows, and the rest was ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U *Bst* DNA Polymerase (NEW ENGLAND Biolabs)
1 X EvaGreen (Biotum)
   a primers:
      1600 nM Mo1aNF (sequence as shown in SEQ ID NO. 4)
      1600 nM Mo1aNR (sequence as shown in SEQ ID NO. 5)
   b primers:
      1600 nM Mo1aNF (sequence as shown in SEQ ID NO. 4)
      1600 nM Mo1aNR (sequence as shown in SEQ ID NO. 5)
      200 nM Mo1aF2 (sequence as shown in SEQ ID NO. 8)
      200 nM Mo1aR2 (sequence as shown in SEQ ID NO. 9)
   c primers:
      1600 nM Mo1aNF (sequence as shown in SEQ ID NO. 4) 1600 nM Mo1aNR (sequence as shown in SEQ ID NO. 5) 800 nM Mo1aFin (sequence as shown in SEQ ID NO. 10) 800 nM Mo1aRin (sequence as shown in SEQ ID NO. 11)
   d primers:
      1600 nM Mo1aNF (sequence as shown in SEQ ID NO. 4)
      1600 nM Mo1aNR (sequence as shown in SEQ ID NO. 5)
      200 nM Mo1aF2 (sequence as shown in SEQ ID NO. 8)
      200 nM Mo1aR2 (sequence as shown in SEQ ID NO. 9)
      800 nM Mo1aFin (sequence as shown in SEQ ID NO. 10)
      800 nM Mo1aRin (sequence as shown in SEQ ID NO. 11)

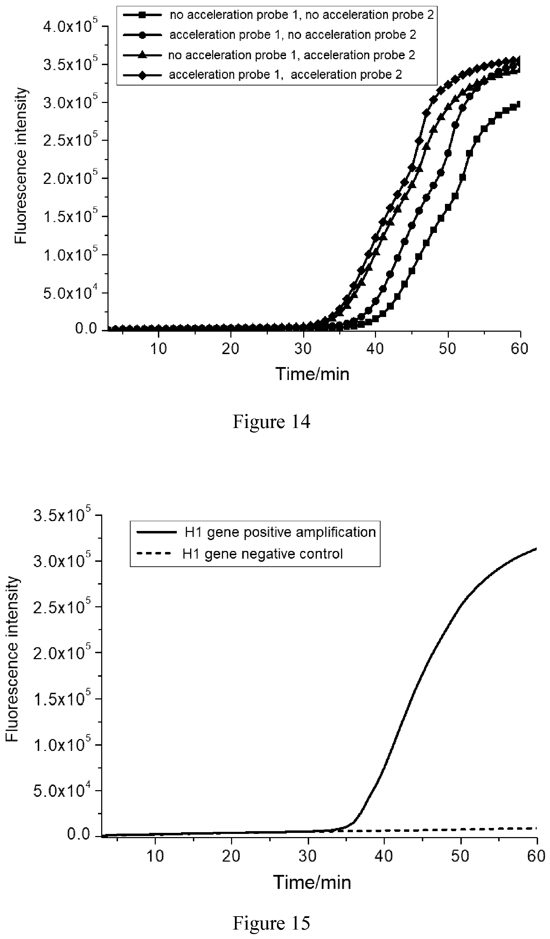

The target nucleic acids corresponding to the primers of each of groups a, b, c, and d are all: MERS-orf1a dsDNA (sequence as shown in SEQ ID NO. 6). ABI StepOne real time PCR reaction temperature was set to be constant at 63°C, and the reaction time was 60 min. The curve of the fluorescence intensity over the reaction time is as shown in Figure 14. Comparing the Ct values of group a and group b, it indicates that acceleration probe 1 plays an acceleration effect; comparing the Ct values of group a and group c, it indicates that acceleration probe 2 plays an acceleration effect; simultaneously comparing the Ct values of group a, group b and group c, it indicates that the acceleration probe 2 plays a better acceleration effect than the acceleration probe 1; and simultaneously comparing the Ct values of group a, group b, group c and group d, it indicates that the acceleration probe 1 and the acceleration probe 2 cooperate with each other to play a synergistic role.

### Example 7 Amplification of influenza A virus HI dsDNA target gene

The A H1N1 virus belongs to the Orthomyxoviridae, Influenza virus A, and the symptoms of influenza A H1N1 are similar to those of a cold, and the patients may have fever, cough, fatigue, and loss of appetite. In 2009, H1N1 was popular in a large area, causing a certain degree of panic. Because the nucleic acid detection of H1N1 is usually carried out by detecting cDNA by PCR after reverse transcription, new primers designed by the method of the present invention can also be applied to the detection of H1N1 virus.

The reaction solution combination was as follows, and the rest was made up with ddH₂O to 25 µL
20 mM Tris-HCl pH 8.8
10 mMKCl
10 mM (NH₄)₂SO₄
14 mM MgSO₄
0.1% Triton X-100
1 M betaine
1.25 mM dNTP
8 U Bst DNA Polymerase (NEW ENGLAND Biolabs)
1 X EvaGreen (Biotum)
Primers:
1600 nM H1-NF (sequence as shown in SEQ ID NO. 12)
1600 nM H1-NR (sequence as shown in SEQ ID NO. 13)

The target nucleic acid is: H1 dsDNA (sequence as shown in SEQ ID NO. 14)

ABI StepOne real time PCR reaction temperature was set to be constant at 63°C, and the reaction time was 60 min. The curve of the fluorescence intensity over the reaction time is as shown in Figure 15. The amplification curve indicates that the method can be applicable to the detection application field of influenza A virus.

### Example 8 Amplification of the Cyprinidae herpes virus III target gene

*The koi herpes* virus disease, herpes virus hematopoietic organ necrosis disease and carp acne sore disease caused by the *Cyprinidae fish herpes* virus infection are serious threats to the Cyprinidae fish culture. The highly pathogenic and highly contagious features of the virus cause the disease to be prevalent worldwide, and the mortality rate of infected fish can reach 80% to 100%. The disease has attracted great attention from the International Organization for Animal Health (OIE) and is listed into a list of key epidemics. At the same time, China has classified the disease as a second-class animal disease and has carried out daily monitoring work. It is very important to develop corresponding detecting techniques to achieve rapid detection of relevant diseases to respond to epidemic situation timely. Therefore, the *Cyprinidae herpes* virus III was selected to serve as a potential application object of the method of the present invention.

The reaction solution combination was as follows, and the rest was made up with ddH₂O to 25 µL
20 mM Tris-HCl pH 8.8
10 mM KCl
10 mM (NH₄)₂SO₄
14 mM MgSO₄
0.1% Triton X-100
1 M betaine
1.25 mM dNTP
8 U *Bst* DNA Polymerase (NEW ENGLAND Biolabs)
1 X EvaGreen (Biotum)
Primers:
1600 nM CyHVIII-NF (sequence as shown in SEQ ID NO. 15)
1600 nM CyHVIII-NR (sequence as shown in SEQ ID NO. 16)

The target nucleic acid is: CyHVIII dsDNA (sequence as shown in SEQ ID NO. 17)

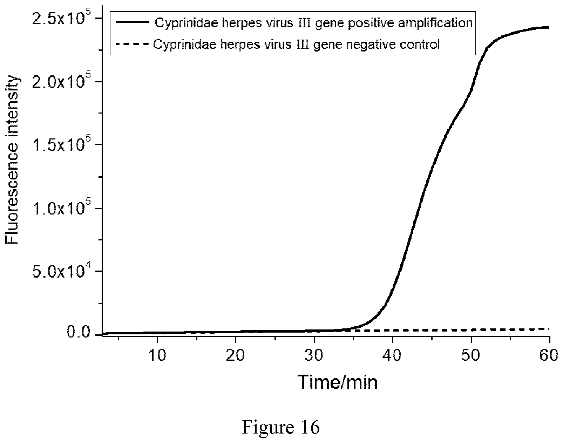

ABI StepOne real time PCR reaction temperature was set to be constant at 63°C, and the reaction time was 60 min. The curve of the fluorescence intensity over the reaction time is as shown in Figure 16. The amplification curve indicates that the method can be applicable to the detection application field of aquatic product prevention and control of *Cyprinidae herpes* virus and so on.
## Claims

1. A method for synthesizing a nucleic acid under constant temperature condition, comprising the steps of:
1) a step of providing a nucleic acid, wherein the nucleic acid has an Nc region that can anneal to an N region on the same strand at each of the 5' end and the 3' end; a loop can be formed when the Nc region at the 3' end anneals to the N region, and the loop comprises an F1c region capable of base pairing; a loop can be formed when the Nc region at the 5' end anneals to the N region, and the loop comprises an R1 region capable of base pairing; and the Nc regions at the 5' end and the 3' end of the nucleic acid have a competitive relationship in annealing to the N region on the same strand;
2) annealing a first oligonucleotide I to the F1c region of the nucleic acid provided in the step 1), and then performing the synthesis step using an F1 region of the first oligonucleotide I as a synthesis starting point; wherein the first oligonucleotide I comprises an N region and the F1 region;
3) using the nucleic acid provided in the step 1) as a template to synthesize its own complementary strand with the 3' end of the Nc region which has annealed to the N region as a synthetic starting point; and referring to the nucleic acid sequence after the synthesis is finished as nucleic acid A;
4) annealing a second oligonucleotide II to an R1c region of the nucleic acid A provided in the step 3), and then performing the synthesis step using an R1 region of the second oligonucleotide II as a synthesis starting point; wherein the second oligonucleotide II comprises the R1 region and an Nc region;
5) using the nucleic acid A provided in the step 3) as a template to synthesize its own complementary strand with the 3' end of the N region which has annealed to the Nc region as a synthetic starting point, and obtaining a nucleic acid strand having a head-to-tail complementary nucleotide sequence on one strand thereof, and the nucleic acid strand having alternately linked complementary nucleotide sequence regions thereon;
wherein the nucleic acid in the step 1) is prepared by the steps of:
1-1) an annealing step, annealing a first oligonucleotide I to an F1c region of the template, wherein the 3' end of the template comprises the F1c region and an N region located at the 5' side of the F1c region, and the 5' end of the template comprises an R1 region, wherein the first oligonucleotide I comprises an N region and an F1 region, and the N region is linked with the 5' side of the F1 region, wherein,
F1 region: a region having a nucleotide sequence complementary to that of the F1c region,
N region: a region of a nucleotide sequence complementary to that of the Nc region;
1-2) synthesizing a first nucleic acid using the F1 region of the first oligonucleotide I as a synthesis starting point; wherein the first nucleic acid has a nucleotide sequence complementary to that of the template, the 5' end of the first nucleic acid has an N region that can anneal to the Nc region on the same strand, and a stem-loop can be formed by annealing the Nc region to the N region;
1-3) displacement obtaining the first nucleic acid synthesized in the step 1-2) by using a polymerase to catalyze a strand displacement reaction;
1-4) an annealing step, annealing a second oligonucleotide II to an R1c region of the first nucleic acid obtained in the step 1-3), wherein the second oligonucleotide II comprises an R1 region and an Nc region, and the Nc region is linked with the 5' side of the R1 region; wherein
R1 region: a region having a nucleotide sequence complementary to that of the R1c region,
Nc region: a region of a nucleotide sequence complementary to that of the N region;
1-5) synthesizing a second nucleic acid using the R1 region of the second oligonucleotide II as a starting point for synthesis; and
1-6) obtaining the nucleic acid in the step 1) by using a polymerase to catalyze a strand displacement reaction to displace the second nucleic acid.

2. The method for synthesizing a nucleic acid under constant temperature condition according to claim 1, it is **characterized in that**, the template in the step 1-1) is RNA, and the first nucleic acid in the step 1-2) is synthesized by an enzyme having reverse transcriptase activity.

3. The method for synthesizing a nucleic acid under constant temperature condition according to any one of claims 1-2, it is **characterized in that**, the nucleic acid fragments of the F1c region, the N region and the R1 region are all 15-60 bp.

4. The method for synthesizing a nucleic acid under constant temperature condition according to claim 1, it is **characterized in that**, the nucleic acid strand obtained is capable of autonomously pairing for infinite extension, and the Nc region at the 3' end on the nucleic acid strand is paired with the complementary segment N region on the strand to act as a synthesis starting point and allow the nucleic acid strand to continuously extend using itself as a template.

5. The method for synthesizing a nucleic acid under constant temperature condition according to claim 1, it is **characterized in that**, in the method for synthesizing a nucleic acid, the nucleic acid amplification is accelerated by a method of introducing the accelerating primer X2 and/or Xin; wherein X2 is a segment located at the 5' side of the R1 region and the F1 region of the complementary strand of the original nucleic acid, and Xin is a middle segment located from the F1c region to the N region and from the Nc region to the R1c region.

## Patentansprüche

1. Ein Verfahren zur Synthese einer Nukleinsäure unter konstanten Temperaturbedingungen, welches die folgenden Schritte umfasst:
1) einen Schritt des Bereitstellens einer Nukleinsäure, wobei die Nukleinsäure eine Nc-Region aufweist, die sich an eine N-Region auf demselben Strang sowohl am 5'-Ende als auch am 3'-Ende anlagern kann; eine Schleife gebildet werden kann, wenn sich die Nc-Region am 3'-Ende an die N-Region anlagert, und die Schleife eine F1c-Region umfasst, die zur Basenpaarung fähig ist; eine Schleife gebildet werden kann, wenn sich die Nc-Region am 5'-Ende an die N-Region anlagert, und die Schleife eine R1-Region umfasst, die zur Basenpaarung fähig ist; und die Nc-Regionen am 5'-Ende und am 3'-Ende der Nukleinsäure eine kompetitive Beziehung beim Anlagern an die N-Region auf dem gleichen Strang aufweisen;
2) Anlagern eines ersten Oligonukleotids I an die F1c-Region der in Schritt 1) bereitgestellten Nukleinsäure, und dann Durchführung des Syntheseschritts unter Verwendung einer F1-Region des ersten Oligonukleotids I als Synthesestartpunkt; wobei das erste Oligonukleotid I eine N-Region und die F1-Region umfasst;
3) Verwenden der in Schritt 1) bereitgestellten Nukleinsäure als Vorlage zur Synthese ihres eigenen komplementären Strangs mit dem 3'-Ende der Nc-Region, die sich an die N-Region als synthetischen Ausgangspunkt angelagert hat; und Bezugnahme auf die Nukleinsäuresequenz nach Beendigung der Synthese als Nukleinsäure A;
4) Anlagern eines zweiten Oligonukleotids II an eine R1c-Region der in Schritt 3) bereitgestellten Nukleinsäure A, und dann Durchführung des Syntheseschritts unter Verwendung einer R1-Region des zweiten Oligonukleotids II als Synthesestartpunkt; wobei das zweite Oligonukleotid II die R1-Region und eine Nc-Region umfasst;
5) Verwendung der in Schritt 3) bereitgestellten Nukleinsäure A als Vorlage zur Synthese ihres eigenen komplementären Strangs mit dem 3'-Ende der N-Region, die sich an die Nc-Region als synthetischen Startpunkt angelagert hat, und Erhalten eines Nukleinsäurestrangs mit einer Kopf-zu-Schwanz komplementären Nukleotidsequenz auf einem Strang, und wobei der Nukleinsäurestrang abwechselnd verbundene komplementäre Nukleotidsequenzregionen aufweist;
wobei die Nukleinsäure in Schritt 1) durch folgende Schritte hergestellt wird:
1-1) ein Anlagerungs-Schritt, bei dem ein erstes Oligonukleotid I an eine F1c-Region der Vorlage angelagert wird, wobei das 3'-Ende der Vorlage die F1c-Region und eine N-Region, die sich auf der 5'-Seite der F1c-Region befindet, umfasst, und das 5'-Ende der Vorlage eine R1-Region umfasst, wobei das erste Oligonukleotid I eine N-Region und eine F1-Region umfasst, und die N-Region mit der 5'-Seite der F1-Region verbunden ist, wobei
F1-Region: eine Region mit einer zu der F1c-Region komplementären Nukleotidsequenz ist,
N-Region: eine Region mit einer zu der Nc-Region komplementären Nukleotidsequenz ist;
1-2) Synthese einer ersten Nukleinsäure unter Verwendung der F1-Region des ersten Oligonukleotids I als Synthesestartpunkt; wobei die erste Nukleinsäure eine zu der Vorlage komplementäre Nukleotidsequenz aufweist, das 5'-Ende der ersten Nukleinsäure eine N-Region hat, die sich an die Nc-Region auf demselben Strang anlagern kann, und eine Haarnadelstruktur durch Anlagern der Nc-Region an die N-Region gebildet werden kann;
1-3) Displacement, wobei die erste in Schritt 1-2) synthetisierte Nukleinsäure unter Verwendung einer Polymerase zur Katalyse einer strand-displacement-Reaktion erhalten wird;
1-4) ein Anlagerungs-Schritt, bei dem ein zweites Oligonukleotid II an eine R1c-Region der ersten in Schritt 1-3) erhaltenen Nukleinsäure angelagert wird, wobei das zweite Oligonukleotid II eine R1-Region und eine Nc-Region umfasst, und die Nc-Region mit der 5'-Seite der R1-Region verbunden ist; wobei
R1-Region: eine Region mit einer zu der R1c-Region komplementären Nukleotidsequenz ist,
Nc-Region: eine Region mit einer zu der N-Region komplementären Nukleotidsequenz ist;
1-5) Synthetisieren einer zweiten Nukleinsäure unter Verwendung der R1-Region des zweiten Oligonukleotids II als Startpunkt für die Synthese; und
1-6) Erhalten der Nukleinsäure in Schritt 1) durch Verwendung einer Polymerase zur Katalyse einer strand-displacement-Reaktion zum Displacement der zweiten Nukleinsäure.

2. Das Verfahren zur Synthese einer Nukleinsäure unter konstanten Temperaturbedingungen nach Anspruch 1, **dadurch gekennzeichnet, dass** die Vorlage in Schritt 1-1) RNA ist, und die erste Nukleinsäure in Schritt 1-2) durch ein Enzym mit reverser Transkriptase-Aktivität synthetisiert wird.

3. Das Verfahren zur Synthese einer Nukleinsäure unter konstanten Temperaturbedingungen nach Ansprüchen 1-2, **dadurch gekennzeichnet, dass** die Nukleinsäurefragmente der F1c-Region, der N-Region und der R1-Region alle 15-60 bp sind.

4. Das Verfahren zur Synthese einer Nukleinsäure unter konstanten Temperaturbedingungen nach Anspruch 1, **dadurch gekennzeichnet, dass** der erhaltene Nukleinsäurestrang in der Lage ist, sich für eine unendliche Verlängerung autonom zu paaren, und dass die Nc-Region am 3'-Ende des Nukleinsäurestrangs mit dem komplementären Segment der N-Region des Strangs gepaart ist, um als Synthesestartpunkt zu wirken und es dem Nukleinsäurestrang zu ermöglichen, sich kontinuierlich zu verlängern, wobei er sich selbst als Vorlage verwendet.

5. Das Verfahren zur Synthese einer Nukleinsäure unter konstanten Temperaturbedingungen nach Anspruch 1, **dadurch gekennzeichnet, dass** in dem Verfahren zur Synthese einer Nukleinsäure die Nukleinsäureamplifikation durch die Methode der Einführung des beschleunigenden Primers X2 und/oder Xin beschleunigt wird; wobei X2 ein Segment ist, das sich an der 5'-Seite der R1-Region und der F1-Region des komplementären Strangs der originalen Nukleinsäure befindet, und Xin ein mittleres Segment ist, das sich von der F1c-Region bis zur N-Region und von der Nc-Region bis zur R1c-Region befindet.

## Revendications

1. Procédé de synthèse d'un acide nucléique dans des conditions de température constante, comprenant les étapes suivantes:
1) une étape de fourniture d'un acide nucléique, où l'acide nucléique comporte une région Nc qui peut s'hybrider avec une région N sur le même brin à chacune de l'extrémité 5' et l'extrémité 3' ; une boucle peut être formée lorsque la région Nc à l'extrémité 3' s'hybride avec la région N, et la boucle comprend une région F1c capable d'appariement de bases ; une boucle peut être formée lorsque la région Nc à l'extrémité 5' s'hybride avec la région N, et la boucle comprend une région R1 capable d'appariement de bases ; et les régions Nc à l'extrémité 5' et l'extrémité 3' de l'acide nucléique ont une relation compétitive pour l'hybridation avec la région N sur le même brin;
2) l'hybridation d'un premier oligonucléotide I à la région F1c de l'acide nucléique fourni dans l'étape 1), puis la conduite de l'étape de synthèse en utilisant une région F1 du premier oligonucléotide I en tant que point de départ de synthèse; où le premier oligonucléotide I comprend une région N et la région F1;
3) l'utilisation de l'acide nucléique fourni dans l'étape 1) en tant que matrice pour synthétiser son propre brin complémentaire avec l'extrémité 3' de la région Nc qui s'est hybridée avec la région N en tant que point de départ de synthèse; et la désignation de la séquence d'acide nucléique une fois que la synthèse est terminée en tant qu'acide nucléique A;
4) l'hybridation d'un deuxième oligonucléotide II avec une région R1c de l'acide nucléique A fourni dans l'étape 3), puis la conduite de l'étape de synthèse en utilisant une région R1 du deuxième oligonucléotide II en tant que point de départ de synthèse; où le deuxième oligonucléotide II comprend la région R1 et une région Nc;
5) l'utilisation de l'acide nucléique A fourni dans l'étape 3) en tant que matrice pour synthétiser son propre brin complémentaire avec l'extrémité 3' de la région N qui s'est hybridée avec la région Nc en tant que point de départ de synthèse, et l'obtention d'un brin d'acide nucléique ayant une séquence nucléotidique complémentaire en tête-à-queue sur un brin de celui-ci, et le brin d'acide nucléique ayant des régions de séquence nucléotidique complémentaires liées en alternance sur celui-ci;
dans lequel l'acide nucléique dans l'étape 1) est préparé par les étapes suivantes:
1-1) une étape d'hybridation, pour hybrider un premier oligonucléotide I avec une région F1c de la matrice, où l'extrémité 3' de la matrice comprend la région F1c et une région N située sur le côté 5' de la région F1c, et l'extrémité 5' de la matrice comprend une région R1, où le premier oligonucléotide I comprend une région N et une région F1, et la région N est liée au côté 5' de la région F1, où,
région F1: une région ayant une séquence nucléotidique complémentaire de celle de la région F1c,
région N: une région d'une séquence nucléotidique complémentaire de celle de la région Nc;
1-2) la synthèse d'un premier acide nucléique en utilisant la région F1 du premier oligonucléotide I en tant que point de départ de synthèse; où le premier acide nucléique a une séquence nucléotidique complémentaire de celle de la matrice, l'extrémité 5' du premier acide nucléique comporte une région N qui peut s'hybrider avec la région Nc sur même brin, et une tige-boucle peut être formée par hybridation de la région Nc avec la région N;
1-3) un déplacement pour obtenir le premier acide nucléique synthétisé dans l'étape 1-2) en utilisant une polymérase pour catalyser une réaction de déplacement de brin;
1-4) une étape d'hybridation, pour hybrider un deuxième oligonucléotide II avec une région R1c du premier acide nucléique obtenu dans l'étape 1-3), où le deuxième oligonucléotide II comprend une région R1 et une région Nc, et la région Nc est liée au côté 5' de la région R1; où
région R1: une région ayant une séquence nucléotidique complémentaire de celle de la région R1c,
région Nc: une région d'une séquence nucléotidique complémentaire de celle de la région N;
1-5) la synthèse d'un deuxième acide nucléique en utilisant la région R1 du deuxième oligonucléotide II en tant que point de départ pour la synthèse; et
1-6) l'obtention de l'acide nucléique dans l'étape 1) en utilisant une polymérase pour catalyser une réaction de déplacement de brin pour déplacer le deuxième acide nucléique.

2. Procédé de synthèse d'un acide nucléique dans des conditions de température constante selon la revendication 1, qui est **caractérisé en ce que** la matrice dans l'étape 1-1) est un ARN et le premier acide nucléique dans l'étape 1-2) est synthétisé par une enzyme ayant une activité transcriptase inverse.

3. Procédé de synthèse d'un acide nucléique dans des conditions de température constante selon l'une quelconque des revendications 1 à 2, qui est **caractérisé en ce que** les fragments d'acide nucléique de la région F1c, la région N et la région R1 sont tous de 15 à 60 pb.

4. Procédé de synthèse d'un acide nucléique dans des conditions de température constante selon la revendication 1, qui est **caractérisé en ce que** le brin d'acide nucléique obtenu est capable d'appariement autonome pour une extension infinie, et la région Nc à l'extrémité 3' sur le brin d'acide nucléique est appariée avec la région N de segment complémentaire sur le brin pour servir de point de départ de synthèse et permettre au brin d'acide nucléique de s'étendre de façon continue en utilisant lui-même en tant que matrice.

5. Procédé de synthèse d'un acide nucléique dans des conditions de température constante selon la revendication 1, qui est **caractérisé en ce que**, dans le procédé de synthèse d'un acide nucléique, l'amplification d'acide nucléique est accélérée par un procédé d'introduction de l'amorce d'accélération X2 et/ou Xin; où X2 est un segment situé sur le côté 5' de la région R1 et la région F1 du brin complémentaire de l'acide nucléique original, et Xin est un segment intermédiaire situé de la région F1c à la région N et de la région Nc à la région R1c.
